(19) Europäisches Patentamt
European Patent Office
Office européen des brevets

(11) EP 3 981 760 B1

(12) **FASCICULE DE BREVET EUROPEEN**

(45) Date de publication et mention de la délivrance du brevet:
**12.04.2023 Bulletin 2023/15**

(21) Numéro de dépôt: **21306254.0**

(22) Date de dépôt: **13.09.2021**

(51) Classification Internationale des Brevets (IPC):
***C07C 303/44*** *(2006.01)* ***C07C 309/15*** *(2006.01)*
***C08F 20/58*** *(2006.01)*

(52) Classification Coopérative des Brevets (CPC):
(C-Sets disponibles)
**C07C 303/44; C07C 309/15; C08F 20/58** (Cont.)

(54) **PROCÉDÉ DE PURIFICATION DE L'ACIDE ACRYLAMIDO-2-MÉTHYL-2-PROPANE SULFONIQUE**

VERFAHREN ZUR REINIGUNG VON ACRYLAMIDO-2-METHYL-2-PROPANSULFONSÄURE

PROCESS FOR THE PURIFICATION OF 2-METHYL-2-ACRYLAMIDO-PROPANESULFONIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.09.2020 FR 2009493**

(43) Date de publication de la demande:
**13.04.2022 Bulletin 2022/15**

(73) Titulaire: **SNF Group**
**42160 Andrézieux-Bouthéon (FR)**

(72) Inventeurs:
- **FAVERO, Cédrick**
  **42160 Andrézieux Bouthéon (FR)**
- **KIEFFER, Johann**
  **42160 Andrézieux Bouthéon (FR)**
- **LEGRAS, Benoît**
  **42160 Andrézieux Bouthéon (FR)**
- **DOUDIN, Raphaël**
  **42160 Andrézieux Bouthéon (FR)**

(74) Mandataire: **Cabinet Laurent & Charras**
**Le Contemporain**
**50 Chemin de la Bruyère**
**69574 Dardilly Cedex (FR)**

(56) Documents cités:
**FR-A1- 3 064 004** **US-A- 4 337 215**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Processed by Luminess, 75001 PARIS (FR)

(Cont. page suivante)

(52) Classification Coopérative des Brevets (CPC):
(Cont.)

C-Sets
**C07C 303/44, C07C 309/15**

## Description

### Domaine technique de l'invention

La présente invention concerne un procédé de purification de l'acide acrylamido-2-méthyl-2-propane sulfonique (ATBS) par distillation en continu.

### Etat de la technique

L'acide acrylamido-2-méthyl-2-propane sulfonique, également appelé ATB S, est un monomère acrylique comportant une fonction acide sulfonique dont la formule est :

[Chem. 1]

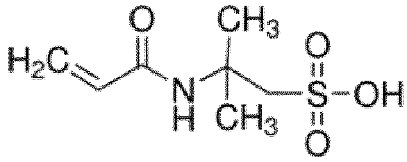

Il est largement utilisé comme additif dans les fibres acryliques, ou encore comme matière première pour obtenir des polymères utilisés en tant que dispersants, épaississants, floculants ou super-absorbants dans divers secteurs comme l'industrie pétrolière, la construction, le textile, le traitement des eaux (dessalement de l'eau de mer, industrie minérale, etc..) ou la cosmétique.

La réaction mise en oeuvre dans le procédé de préparation de l'acide acrylamido-2-méthyl-2-propane sulfonique répond au schéma réactionnel ci-dessous, dans lequel l'acrylonitrile est présent en excès de manière à être à la fois le solvant de la réaction et un réactif. L'acrylonitrile est mis en contact avec de l'acide sulfurique fumant (oleum) et de l'isobutylène.

[Chem. 2]

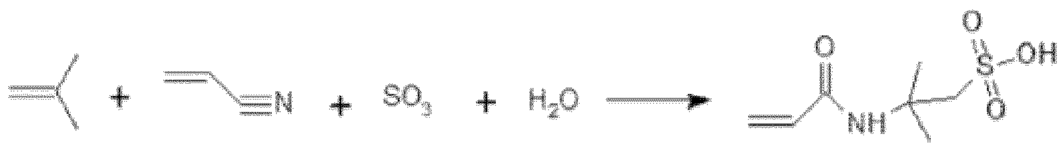

L'acide acrylamido-2-méthyl-2-propane sulfonique n'est pas soluble dans le solvant acrylonitrile. En conséquence, le produit de réaction est sous une forme de suspension de cristaux dans le solvant de réaction.

A titre d'exemples, les documents US 6,448,347 et CN 102351744 décrivent un procédé de fabrication de l'acide acrylamido-2-méthyl-2-propane sulfonique selon un mode continu.

L'acide acrylamido-2-méthyl-2-propane sulfonique est par la suite séparé de l'acrylonitrile, généralement par filtration, puis séché. Le séchage de l'acide acrylamido-2-méthyl-2-propane sulfonique est nécessaire afin de diminuer la quantité d'acrylonitrile et d'acrylamide restant présents dans le cristal. En effet, ces deux composés étant classifiés comme composants cancérigènes CMR, il est nécessaire de procéder à une filtration efficace puis à un séchage, afin d'obtenir des teneurs de ces deux composés les plus faibles possible.

Bien souvent, une étape supplémentaire de purification est nécessaire car des impuretés, même à faibles concentrations, affectent fortement la polymérisation, ainsi que la qualité du polymère obtenu, plus particulièrement son poids moléculaire et le taux d'insoluble dans l'eau.

Ainsi, le document WO 2009/072480, qui porte sur un procédé de fabrication d'acide acrylamido-2-méthyl-2-propane sulfonique, explique que les impuretés de type acide 2-méthyl-2-propényl-sulfonique (IBSA) et acide 2-méthylidène-1,3- propylènedisulfonique (IBDSA) affectent fortement la polymérisation au-delà d'une certaine concentration.

Il existe de nombreuses méthodes de purification de l'acide acrylamido-2-méthyl-2-propane sulfonique. Le plus souvent, l'acide acrylamido-2-méthyl-2-propane sulfonique est redissous dans un solvant à chaud, afin d'obtenir une solution saturée. Lors du refroidissement, des cristaux de haute pureté sont obtenus.

Les cristaux ainsi obtenus sont ensuite séchés sous vide afin d'améliorer un peu plus leur pureté en éliminant le solvant résiduel.

Le document US 4,337,215 décrit une méthode de purification de l'acide acrylamido-2-méthyl-2-propane sulfonique par recristallisation dans de l'acide acétique, par dissolution à chaud et cristallisation par rampe de refroidissement. Malgré la bonne pureté de l'acide acrylamido-2-méthyl-2-propane sulfonique obtenu, le procédé, dont le rendement est limité, fait intervenir de multiples étapes de dissolution/refroidissement et requiert un traitement de l'acide acétique usagé pour le régénérer par distillation avant réutilisation dans un nouveau lot (« batch » en anglais) de recristallisation de l'acide acrylamido-2-méthyl-2-propane sulfonique. Le procédé décrit est de type « batch », c'est-à-dire en mode discontinu.

Le document CN 103664709 décrit un procédé de préparation d'acide acrylamido-2-méthyl-2-propane sulfonique permettant de s'affranchir de cette étape de séchage longue et coûteuse. L'étape de séchage est substituée par une étape de lavage à l'acide acétique glacial dans lequel l'ATBS n'est pas soluble. Bien que ce procédé permette de raccourcir la durée de la synthèse d'acide acrylamido-2-méthyl-2-propane sulfonique, la consommation de solvant et l'énergie thermique nécessaire à la purification par recristallisation sont encore trop importantes. Le procédé décrit est de type « batch ».

Tous ces procédés utilisent une solution saturée d'acide acrylamido-2-méthyl-2-propane sulfonique dissoute dans un solvant nécessitant une étape de dissolution des cristaux à chaud, ce qui ajoute une étape fortement consommatrice d'énergie et pose différents problèmes liés à l'utilisation de solvant (risque lors de la manipulation, transport et stockage du solvant, dégradation du matériel utilisé liée à la nature du solvant, empreinte environnementale).

Dans le contexte actuel, il existe un besoin de développer des nouveaux procédés respectant les normes QHSE (Qualité, Hygiène, Sécurité et Environnement).

La politique Qualité, Hygiène, Sécurité, Environnement (QHSE) est un domaine d'expertise comprenant l'identification et le respect des normes de production d'une entreprise, avec une attention particulière à l'environnement de travail des salariés, au matériel et au respect de l'environnement :

- Qualité : Maintien de la bonne qualité des produits proposés.
- Hygiène et Sécurité : Diminution des risques pour les salariés et les installations.
- Environnement : Procédés plus écologique avec un impact environnemental réduit.

La demanderesse a découvert de manière inattendue que les problèmes précédemment exposés pouvaient être résolus grâce à un procédé de purification de l'acide acrylamido-2-méthyl-2-propane sulfonique réalisé en continu par distillation d'une solution aqueuse d'acide acrylamido-2-méthyl-2-propane sulfonique, à pression réduite.

Un objet de la présente invention est de fournir un nouveau procédé de purification de l'acide acrylamido-2-méthyl-2-propane sulfonique répondant aux attentes QHSE.

Le procédé selon l'invention permet d'obtenir des cristaux d'acide de très bonne qualité, tout en diminuant les risques liés à la manipulation de solvants toxiques et en réduisant l'impact environnemental du procédé de purification de l'acide acrylamido-2-méthyl-2-propane sulfonique.

**Exposé de l'invention**

La présente invention concerne un procédé de purification de l'acide acrylamido-2-méthyl-2-propane sulfonique par distillation en continu.

La présente invention concerne également un procédé de fabrication d'acide 2-acrylamido-2-méthylpropane sulfonique (ATBS) comprenant une étape de purification dudit acide acrylamido-2-méthyl-2-propane sulfonique par le procédé selon l'invention.

La présente invention concerne également l'utilisation des cristaux de l'acide acrylamido-2-méthyl-2-propane sulfonique obtenus selon le procédé de l'invention pour la préparation d'un sel ou d'une solution de sels d'acide acrylamido-2-méthyl-2-propane sulfonique.

La présente invention concerne également l'utilisation des cristaux de l'acide acrylamido-2-méthyl-2-propane sulfonique obtenus selon le procédé de l'invention, pour la fabrication de polymères.

La demande divulgue des polymères obtenus à partir d'acide acrylamido-2-méthyl-2-propane sulfonique ou de ses sels, obtenus à partir de cristaux d'acide acrylamido-2-méthyl-2-propane sulfonique ou de ses sels obtenus selon le procédé de l'invention, ainsi que l'utilisation de ces polymères dans la récupération du pétrole et du gaz, dans le traitement de l'eau, dans le traitement des boues, dans la fabrication du papier, dans la construction, dans l'industrie minière, dans la formulation de produits cosmétiques, dans la formulation de détergents, dans la fabrication du textile, ou dans l'agriculture.

Le procédé selon l'invention peut être incorporé dans tous les procédés de préparation de l'acide acrylamido-2-méthyl-2-propane sulfonique déjà existants.

***Procédé de purification de l'acide acrylamido-2-méthyl-2-propane sulfonique***

La présente invention a pour objet un procédé de purification de l'acide acrylamido-2-méthyl-2-propane sulfonique comprenant les étapes successives suivantes :

1) préparation d'une suspension de cristaux d'acide acrylamido-2-méthyl-2-propane sulfonique par distillation d'une solution aqueuse d'acide acrylamido-2-méthyl-2-propane sulfonique,
2) isolation des cristaux d'acide acrylamido-2-méthyl-2-propane sulfonique par séparation solide/liquide de ladite suspension,

caractérisé en ce que l'étape de distillation se fait en continu et à une pression inférieure à la pression atmosphérique.
Comme son nom l'indique, l'acide acrylamido-2-méthyl-2-propane sulfonique purifié selon l'invention est sous forme acide.
Par « étapes successives », on désigne des étapes qui se suivent chronologiquement. En d'autres termes, des étapes successives sont réalisées dans l'ordre indiqué et ne peuvent pas être interverties. En revanche, une ou plusieurs étapes intermédiaires peuvent, éventuellement, être intercalées entre deux étapes successives.
Par « solution aqueuse », on entend une solution à base d'eau qui, de manière générale, ne comporte pas de particules solides d'acide acrylamido-2-méthyl-2-propane sulfonique. Néanmoins, il est possible qu'une quantité faible de particules solides d'acide acrylamido-2-méthyl-2-propane sulfonique soit présente dans ladite solution. Par « quantité faible », on entend moins de 5%, préférentiellement moins de 2%, encore plus préférentiellement moins de 1 % par rapport à la masse de la solution aqueuse. De préférence, la solution d'acide acrylamido-2-méthyl-2-propane sulfonique ne contient pas de particule solide d'acide acrylamido-2-méthyl-2-propane sulfonique.
De manière générale, les bornes des plages de valeurs indiquées ci-après peuvent être combinées selon l'invention. Ainsi deux plages de valeur définies par une seule borne définissent également la plage de valeur délimitée par ces deux bornes.

**Description sommaire de la figure**

[Fig. 1] La figure 1 est une vue schématique du procédé selon l'invention (en trait plein), avec ses différentes variantes (en traits en pointillés).
Bien entendu, les dimensions et les proportions des éléments illustrés sur la figure 1 ont pu être exagérées par rapport à la réalité, et n'ont été données que dans le but de faciliter la compréhension de l'invention.

**Description détaillée**

La figure 1 reprend les étapes du procédé selon l'invention, en traits pleins pour les étapes obligatoires, et en traits en pointillés pour les étapes optionnelles. Les flux de circulation de fluides sont représentés de la même façon, c'est-à-dire soit en traits pleins, soit en traits en pointillés.
Selon le procédé de l'invention, un flux 1 d'ATBS en solution aqueuse est traité dans l'étape 1), généralement par un dispositif de distillation. Cette solution aqueuse est soit issue d'une pré-étape optionnelle de préparation d'une solution aqueuse d'ATBS, soit fournie telle quelle. Il sort de l'étape 1) un flux 2 d'une suspension de cristaux d'ATBS, et un flux 3 de solvant distillé. L'étape 2 de séparation solide liquide est effectuée par exemple au moyen d'une centrifugeuse ou d'un filtre clos de type Nutsche.
Ces étapes ainsi que les étapes optionnelles seront détaillées ci-après, en référence avec la figure 1.

***Pré-étape - Préparation d'une solution aqueuse d'acide acrylamido-2-méthyl-2-propane sulfonique (optionnelle)***

D'ordinaire, un procédé de purification requiert la dissolution à chaud du produit à purifier dans un solvant afin d'obtenir une solution saturée qui, lors d'une étape de refroidissement de la solution, favorise la formation de cristaux.
Dans le cas de l'acide acrylamido-2-méthyl-2-propane sulfonique, les solvants classiquement utilisés sont avantageusement l'acide acétique, l'acrylonitrile et de façon générale tous les solvants classiquement utilisés dans les purifications et ayant de 1 à 10 atomes de carbone par molécule de solvant (c'est-à-dire le plus souvent : alcools, amides, cétones, aldéhydes, éthers, acides carboxyliques, alcanes, esters, nitriles, hydrocarbures halogénés etc....), et leurs mélanges.
De tels solvants posent différents problèmes liés à leur utilisation, que ce soient les risques liés à leur manipulation, les risques lors de leur transport et leur stockage ou les risques de dégradation du matériel utilisé (notamment dans le cas des acides).
Grâce au procédé selon l'invention, il est possible d'utiliser majoritairement de l'eau comme solvant lors de la

purification d'acide acrylamido-2-méthyl-2-propane sulfonique.

Selon un mode particulier de l'invention, la solution aqueuse d'acide acrylamido-2-méthyl-2-propane sulfonique, préalablement à la distillation, comprend au moins 80% massique d'eau, préférentiellement au moins 85% massique, plus préférentiellement au moins 90% massique, plus préférentiellement au moins 92% massique plus préférentiellement au moins 95% massique, et encore plus préférentiellement au moins 99% massique, par rapport à la masse totale des solvants de la solution. Préférentiellement, elle comprend 100% massique d'eau par rapport à la masse totale des solvants de la solution.

La solution aqueuse d'acide acrylamido-2-méthyl-2-propane sulfonique est par exemple obtenue par dissolution de l'acide acrylamido-2-méthyl-2-propane sulfonique dans une solution aqueuse. Cette dissolution peut être effectuée dans un mélangeur. A titre d'exemple et de manière non limitative, un tel mélangeur peut être choisi parmi les réacteurs avec agitateurs, les réacteurs boucles, les mélangeurs statiques, les microréacteurs, et les réacteurs pistons.

Il est possible que la solution aqueuse d'acide acrylamido-2-méthyl-2-propane sulfonique utilisée dans le procédé selon l'invention comprenne d'autre(s) solvant(s). Ce(s) solvant(s) peu(ven)t, à titre d'exemple et de manière non limitative, provenir d'impuretés présentes dans l'acide acrylamido-2-méthyl-2-propane sulfonique à purifier.

Avantageusement, la solution aqueuse d'acide acrylamido-2-méthyl-2-propane sulfonique préparée contient moins de 20% massique d'autre(s) solvant(s) que l'eau, préférentiellement moins de 15% massique, plus préférentiellement moins de 10% massique, plus préférentiellement moins de 8% massique, encore plus préférentiellement moins de 5% massique, et encore plus préférentiellement moins de 1% massique, par rapport à la masse total des solvants de la solution. Préférentiellement, ladite solution aqueuse ne contient pas d'autre solvant que l'eau.

La dissolution peut se faire à chaud (typiquement à plus de 50°C), à froid (typiquement à moins de 10°C), ou à température ambiante, c'est-à-dire entre 10 et 40°C, préférentiellement entre 10 et 30°C. Avantageusement, la dissolution de l'acide acrylamido-2-méthyl-2-propane sulfonique se fait à température ambiante.

Les monomères de la solution aqueuse d'acide acrylamido-2-méthyl-2-propane sulfonique sont généralement entièrement sous forme acide, c'est-à-dire qu'ils ne sont pas neutralisés sous forme de sels d'acide acrylamido-2-méthyl-2-propane sulfonique. S'il y a des sels d'acide acrylamido-2-méthyl-2-propane sulfonique dans ladite solution aqueuse, ils sont présents typiquement à moins de 1%, par rapport au total des monomères d'acide acrylamido-2-méthyl-2-propane sulfonique de la solution.

La concentration en acide acrylamido-2-méthyl-2-propane sulfonique de la solution aqueuse d'acide acrylamido-2-méthyl-2-propane sulfonique distillée à l'étape 1) est généralement comprise entre 10% en masse (par rapport à la masse de la solution aqueuse) et le pourcentage conduisant à la saturation de la solution, appelé ici saturation de la solution. Préférentiellement, elle est comprise entre 20% en masse et la saturation de la solution, plus préférentiellement entre 30% en masse et la saturation de la solution. Dans un mode particulier, la solution d'acide acrylamido-2-méthyl-2-propane sulfonique est à saturation.

La valeur de saturation de la solution aqueuse d'acide acrylamido-2-méthyl-propane sulfonique dépend de la température. Ainsi, à 25°C par exemple, la saturation de la solution est de 58% en masse. La personne du métier saura ajuster la concentration de la solution d'acide acrylamido-2-méthyl-2-propane sulfonique en fonction de la température pour arriver à une solution saturée.

Le procédé selon l'invention fonctionne également avec une solution dont la concentration est faible, c'est-à-dire inférieure à 10% en masse d'ATBS, quelle que soit la température. Cependant une telle concentration n'est pas intéressante du point de vue industriel. En effet, une concentration faible nécessite la distillation d'une quantité d'eau plus importante, ce qui augmente la consommation énergétique et réduit la productivité et la rentabilité du procédé de purification.

Dans les procédés de purification conventionnels, au moins un inhibiteur de polymérisation est utilisé classiquement afin d'éviter un risque de polymérisation des monomères lors de leur purification. Le procédé de l'invention permet de réduire la quantité de cet (ces) inhibiteur(s) de polymérisation, voire de s'en passer. En effet, lesdits inhibiteurs de polymérisation pouvant avoir un effet négatif lors de la polymérisation, il est intéressant de pouvoir réduire leur quantité.

Lorsque la solution aqueuse comprend au moins un inhibiteur de polymérisation, celui-ci est avantageusement choisi parmi, par exemple et de façon non limitative, le phénotiazine, le (2,2,6,6-tétraméthylpipéridin-1-yl)oxyl, le 4-hydroxy-2,2,6,6-tétraméthylpipéridin-1-oxyl, les dérivés de phénylène diamines, et leurs mélanges. Préférentiellement, l'inhibiteur de polymérisation est le 4-hydroxy-2,2,6,6-tétraméthylpipéridin-1-oxyl.

Les inhibiteurs de polymérisation peuvent être également de type dérivés phénoliques, comme l'hydroquinone ou le paraméthoxyphénol, bien que ceux-ci fonctionnent moins bien que les inhibiteurs de polymérisation décrits ci-dessus. En effet, ces inhibiteurs de type dérivés phénoliques ont besoin d'oxygène dissous dans la solution pour jouer efficacement leur rôle d'inhibiteur de polymérisation. La distillation de la solution aqueuse d'acide acrylamido-2-méthyl-2-propane sulfonique, se faisant en continu et à une pression inférieure à la pression atmosphérique, il y a moins d'oxygène dissous pour assurer le bon fonctionnement de ces inhibiteurs. Lorsque le procédé selon l'invention comprend au moins un inhibiteur de polymérisation il n'est préférentiellement pas choisi parmi les inhibiteurs de type dérivés phénoliques.

L'inhibiteur de polymérisation peut être déjà présent dans l'acide acrylamido-2-méthyl-2-propane sulfonique ou dans la solution aqueuse d'acide acrylamido-2-méthyl-2-propane sulfonique, ou il peut être rajouté au moment de la formation de ladite solution aqueuse, par exemple par dissolution, ou encore il peut être rajouté de façon continue lors de l'étape de distillation.

Lorsque la solution aqueuse d'acide acrylamido-2-méthyl-2-propane sulfonique contient un inhibiteur de polymérisation, sa quantité est généralement inférieure à 1%, préférentiellement inférieure à 0,1%, plus préférentiellement inférieure à 0,01%, et encore plus préférentiellement inférieure à 0,001%, en masse par rapport à la quantité d'acide acrylamido-2-méthyl-2-propane sulfonique. Préférentiellement, la solution aqueuse d'acide acrylamido-2-méthyl-2-propane sulfonique ne comprend pas d'inhibiteur de polymérisation.

Classiquement, la recristallisation de l'acide acrylamido-2-méthyl-2-propane sulfonique nécessite une étape de dissolution à chaud afin d'avoir une solution saturée et de forcer la cristallisation lors d'une étape lente de refroidissement de ladite solution. Ces étapes sont fortement consommatrices en énergie.

Dans un mode préféré, le procédé selon l'invention ne nécessite pas d'étape de dissolution à chaud suivi d'une étape lente de refroidissement.

La préparation de la solution aqueuse d'acide acrylamido-2-méthyl-2-propane sulfonique peut être réalisée de manière discontinue (en « batch ») ou continue. Préférentiellement elle est réalisée en continu.

***Etape 1) - Distillation de la solution aqueuse d'acide acrylamido-2-méthyl-2-propane sulfonique***

Le procédé selon l'invention comprend une étape de distillation en continu de la solution aqueuse d'acide acrylamido-2-méthyl-2-propane sulfonique fournie telle quelle ou bien préparée en pré-étape décrite ci-dessus.

L'étape 1) de distillation selon l'invention se fait en continu et à une pression inférieure à la pression atmosphérique, généralement dans un dispositif de distillation sous vide, qui est typiquement un évaporateur. Elle est donc également qualifiée ici de « distillation sous vide ».

Par « procédé continu » selon l'invention, on entend un procédé dans lequel au moins un flux entre continuellement, avantageusement dans un dispositif de distillation sous vide, et d'où au moins un flux sort sans interruption. Le procédé continu selon l'invention peut fonctionner pendant plusieurs jours, voire plusieurs mois sans interruption.

Il est cependant possible que le procédé continu selon l'invention soit exceptionnellement interrompu puis relancé pour diverses raisons. A titre d'exemple et de manière non limitative, nous pouvons citer une opération de maintenance, ou un problème technique. Le procédé est tout de même considéré comme continu, par opposition à un procédé en mode discontinu (« batch » ou semi « batch ») qui s'effectue avec au moins un arrêt entre chaque lot.

Lorsque la solution d'acide acrylamido-2-méthyl-2-propane sulfonique est distillée, typiquement par passage dans un évaporateur, des cristaux d'acide acrylamido-2-méthyl-2-propane sulfonique commencent à se former. Il y a alors coexistence de la solution aqueuse d'acide acrylamido-2-méthyl-2-propane sulfonique sur laquelle s'effectue la distillation et des particules solides d'acide acrylamido-2-méthyl-2-propane sulfonique.

La distillation sous vide peut se faire à l'aide d'un évaporateur. Il peut s'agir d'un évaporateur à film tombant, ou d'un évaporateur à film montant, ou d'un évaporateur à film mince raclé, ou d'un évaporateur court trajet, ou d'un évaporateur à circulation forcé, ou d'un évaporateur à tube spiralé, ou encore d'un évaporateur par refroidissement flash. Il peut également s'agir d'un réacteur agité continu. Préférentiellement, la distillation se fait dans un évaporateur à film mince raclé, ou un évaporateur court trajet, ou un évaporateur à circulation forcée. Encore plus préférentiellement, la distillation se fait dans un évaporateur à film mince raclé.

De manière générale, un évaporateur est un dispositif comprenant une entrée de solution à traiter (solution aqueuse d'ATBS), une sortie pour évacuer l'eau et le(s) éventuel(s) solvant(s) distillés et une sortie pour évacuer la solution concentrée (ou suspension en présence de cristaux et/ou de particules). Par « X et/ou Y », on entend selon l'invention soit X, soit Y, soit X et Y.

Le temps de résidence de la solution aqueuse d'acide acrylamido-2-méthyl-2-propane sulfonique dans le dispositif de distillation, qui est avantageusement un évaporateur, autrement dit la durée de distillation, est avantageusement compris entre 1 seconde et 600 secondes, préférentiellement entre 3 secondes et 300 secondes, plus préférentiellement entre 30 secondes et 100 secondes. Le temps de résidence correspond à la durée nécessaire à la réalisation de l'étape 1), c'est-à-dire à la durée de la préparation de la suspension de cristaux d'ATBS par distillation de la solution aqueuse d'ATBS. En d'autres termes, dans le cas d'un évaporateur, il s'agit du temps de séjour de l'ATBS, entre l'entrée et la sortie du dispositif.

La distillation peut être effectuée dans un évaporateur vertical ou horizontal. Préférentiellement elle est effectuée dans un évaporateur vertical.

La solution aqueuse d'acide acrylamido-2-méthyl-2-propane sulfonique peut circuler à co-courant ou à contre-courant par rapport aux vapeurs générées par l'évaporation. Préférentiellement, elle circule à contre-courant des vapeurs dans le dispositif de distillation. En d'autres termes, la solution aqueuse d'ATBS est introduite dans le dispositif de distillation, avantageusement un évaporateur, à co-courant ou à contre-courant par rapport au solvant distillé.

La solution aqueuse d'acide acrylamido-2-méthyl-2-propane sulfonique peut circuler dans un ou plusieurs évaporateurs en série. Préférentiellement elle circule dans un évaporateur unique.

Selon le procédé de l'invention, si l'on se réfère à la figure 1, le flux entrant dans le dispositif de distillation de l'étape 1) est le flux 1 qui correspond à la solution aqueuse d'acide acrylamido-2-méthyl-2-propane sulfonique fournie ou bien préparée à la pré-étape.

Le débit du flux entrant 1 est avantageusement compris entre 100 kg/h et 200 000 kg/h, plus avantageusement entre 200 et 100 000 kg/h.

Les flux sortants du dispositif de distillation de l'étape 1) sont le flux 2 qui correspond à une suspension de cristaux d'acide acrylamido-2-méthyl-2-propane sulfonique obtenue après distillation sous vide du flux 1, et le flux 3 qui correspond au solvant distillé durant la distillation.

Le ratio massique entre le flux 2 et le flux 3 est généralement compris entre 0,01 et 200, préférentiellement entre 0,1 et 10, plus préférentiellement entre 1 et 5.

Dans le flux 2, la proportion d'acide acrylamido-2-méthyl-2-propane sulfonique sous forme solide (cristaux) et liquide (solubilisé) représente généralement entre 50% et 95% en masse, préférentiellement entre 60% et 80% par rapport à la masse total du flux 2. Le reste des composants du flux 2 est principalement de l'eau et potentiellement un (ou d') autre(s) solvant(s).

Le flux 3 contient principalement de l'eau et peut minoritairement contenir un (ou d') autre(s) solvant(s), ou de l'acide acrylamido-2-méthyl-2-propane sulfonique.

Le débit d'évaporation dépend du débit du flux entrant et de la surface de contact entre le fluide entrant et le dispositif de distillation, de la température d'un fluide caloporteur le plus souvent utilisé en relation avec le dispositif de distillation, et enfin de la pression dans l'évaporateur. La surface de contact est dépendante de la taille du dispositif de distillation ; elle est définie comme étant la surface interne du dispositif de distillation en contact avec la solution aqueuse de l'acide acrylamido-2-méthyl-2-propane sulfonique et/ou avec la suspension d'acide acrylamido-2-méthyl-2-propane sulfonique formée à l'intérieur du dispositif de distillation lors de l'étape 1) de distillation.

Le débit d'évaporation est calculé de la manière suivante :

$$\text{Débit d'évaporation } (kg/h/m^2) = \text{débit flux 3 } (kg/h) \, / \, \text{surface du dispositif de distillation } (m^2)$$

Le débit d'évaporation est avantageusement compris entre 10 kg/h/$m^2$ et 4000 kg/h/$m^2$, préférentiellement entre 20 et 1000 kg/h/$m^2$, encore plus préférentiellement entre 30 et 100 kg/h/$m^2$.

Pour faciliter l'évaporation du solvant, la distillation peut se faire à chaud. Le chauffage durant la distillation peut se faire par diverses technologies. A titre d'exemple et de manière non limitative, nous pouvons citer le chauffage avec de la vapeur, à l'eau chaude, par électricité, par compression de vapeur, ou encore à l'aide d'une pompe à chaleur. Ainsi, le dispositif de distillation peut être de type double paroi, un fluide caloporteur chaud circulant entre les deux parois.

La température de la paroi interne du dispositif de distillation est avantageusement comprise entre 5 et 90°C, préférentiellement entre 30 et 80°C.

La température de la solution aqueuse d'acide acrylamido-2-méthyl-2-propane sulfonique distillée (c'est-à-dire à l'intérieur du dispositif de distillation) est généralement comprise entre 5 et 90°C, préférentiellement entre 25 et 70°C.

La pression lors de la distillation est avantageusement comprise entre 1 et moins de 1000 mbar absolu (1 mbar = 100 Pa). Elle est préférentiellement inférieure à 900 mbar absolu, plus préférentiellement inférieure à 800 mbar absolu, plus préférentiellement inférieure à 700 mbar absolu, plus préférentiellement inférieure à 600 mbar absolu, plus préférentiellement inférieure à 500 mbar absolu, plus préférentiellement inférieure à 400 mbar absolu, plus préférentiellement inférieure à 300 mbar absolu, plus préférentiellement inférieure à 200 mbar absolu, plus préférentiellement inférieure à 100 mbar absolu et encore plus préférentiellement inférieure à 50 mbar absolu, et avantageusement supérieure à 1 mbar absolu. La pression absolue correspond à la pression par rapport à la pression zéro (vide).

Le pH de la solution aqueuse sur laquelle est effectuée la distillation, est généralement et de préférence inférieur à 2. Préférentiellement, la distillation sous vide se fait sur la forme acide des monomères d'acide acrylamido-2-méthyl-2-propane sulfonique et non sur la forme partiellement ou totalement neutralisée desdits monomères.

Selon un autre mode particulier de l'invention, le flux 2 peut être refroidi avant l'étape 2) de séparation solide/liquide de la suspension d'acide acrylamido-2-méthyl-2-propane sulfonique. Cela a pour effet d'augmenter la productivité et la rentabilité du procédé de l'invention en accélérant la cristallisation de l'acide acrylamido-2-méthyl-2-propane sulfonique.

Le flux 2 peut être refroidi à l'aide, par exemple et de façon non limitative, d'un échangeur de chaleur.

Selon un mode particulier de l'invention, illustré sur la figure 1, le flux 3 peut être recyclé partiellement ou totalement dans la pré-étape si une telle pré-étape est présente, avec ou sans étape de traitement préalable. En d'autres termes, le solvant distillé issu de l'étape 2) est recyclé au moins partiellement dans la solution aqueuse, généralement soit dans la pré-étape, soit par mélange avec ladite solution aqueuse avant l'étape 1).

Selon un autre mode particulier de l'invention, illustré sur la figure 1, le flux 3 peut être recyclé partiellement ou totalement, généralement pour laver les cristaux d'acide acrylamido-2-méthyl-2-propane sulfonique obtenus après l'étape 2) de séparation liquide/solide, avec et/ou sans étape de traitement préalable.

Selon un autre mode particulier de l'invention, illustré sur la figure 1, le flux 3 peut être utilisé partiellement ou totalement pour préparer un sel d'acide d'acrylamido-2-méthyl-2-propane sulfonique, avec ou sans étape de traitement préalable. Ce sel est généralement ensuite utilisé pour la synthèse de polymères.

***Etape 2) - Séparation solide/liquide de la suspension d'acide acrylamido-2-méthyl-2-propane sulfonique obtenue après distillation***

Les cristaux d'acide acrylamido-2-méthyl-2-propane sulfonique sont isolés de préférence par une étape de séparation liquide/solide du flux 2 le plus souvent conduisant à l'obtention sous forme d'un gâteau de cristaux d'acide acrylamido-2-méthyl-2-propane sulfonique. Cette étape est réalisée, à titre d'exemple et de manière non limitative, grâce à une centrifugeuse horizontale ou verticale, un décanteur, un filtre presse, un filtre à bande, un filtre à disques, un filtre clos sous vide, un filtre clos sous pression ou un filtre à tambour rotatif. Préférentiellement, la séparation liquide/solide se fait à l'aide d'une centrifugeuse ou d'un filtre clos de type Nutsche.

La solution aqueuse récupérée après l'étape 2) de séparation liquide/solide contient principalement de l'eau et de l'acide acrylamido-2-méthyl-2-propane sulfonique solubilisé, et peut minoritairement contenir un (ou des) autre(s) solvant(s) ou une (ou des) impureté(s).

Selon un mode particulier de l'invention, illustré sur la figure 1, la solution aqueuse récupérée après la séparation liquide/solide peut être recyclée partiellement ou totalement dans la pré-étape si une telle pré-étape est présente, avec ou sans étape de traitement préalable.

Selon un mode particulier de l'invention, illustré sur la figure 1, la solution aqueuse récupérée après la séparation liquide/solide peut être recyclée partiellement ou totalement dans l'étape 1), avec ou sans étape de traitement préalable, directement dans le dispositif de distillation ou ajoutée à la solution aqueuse d'acide acrylamido-2-méthyl-2-propane sulfonique.

Selon un autre mode particulier de l'invention, illustré sur la figure 1, la solution aqueuse récupérée après la séparation liquide/solide peut être recyclée partiellement ou totalement pour préparer un sel d'acide d'acrylamido-2-méthyl-2-propane sulfonique, avec ou sans étape de traitement préalable.

La séparation liquide/solide peut être réalisée de manière discontinue (en lot ou « batch ») ou continue. Préférentiellement elle est réalisée en continu.

***Lavage des cristaux d'acide acrylamido-2-méthyl-2-propane sulfonique (optionnel)***

Lors d'une étape optionnelle de lavage illustrée sur la figure 1, les cristaux d'acide acrylamido-2-méthyl-2-propane sulfonique obtenus à l'étape 2) de séparation solide/liquide peuvent être lavés à l'aide d'au moins une solution de lavage.

La solution de lavage comprend essentiellement de l'eau. Elle peut néanmoins comprendre d'autre(s) solvant(s), ainsi que de l'acide acrylamido-2-méthyl-2-propane sulfonique dissous.

Avantageusement, la solution de lavage ne contient pas plus de 20% en masse d'autre(s) solvant(s) que l'eau par rapport à la masse totale de la solution de lavage, préférentiellement moins de 15% en masse, plus préférentiellement moins de 10% en masse, encore plus préférentiellement moins de 8% en masse, encore plus préférentiellement moins de 5% en masse, et encore plus préférentiellement moins de 1% en masse.

Selon un mode particulier de l'invention, le lavage des cristaux obtenus à l'issue de la distillation sous vide peut s'effectuer en pulvérisant de la solution de lavage sur lesdits cristaux.

Selon un autre mode particulier de l'invention, le lavage des cristaux obtenus à l'issue de la distillation sous vide peut s'effectuer en mettant en suspension les cristaux dans la solution de lavage.

Selon un autre mode particulier de l'invention, illustré sur la figure 1, la solution de lavage est, partiellement ou totalement, la phase distillée obtenue à l'étape 1) correspondant au flux 3.

Le ratio massique entre la solution de lavage et les cristaux d'acide acrylamido-2-méthyl-2-propane sulfonique obtenus à l'issue de la distillation sous vide est avantageusement compris entre 0,1:1 et 10:1 (solution de lavage/cristaux), plus préférentiellement entre 0.2:1 et 5:1.

Les cristaux d'acide acrylamido-2-méthyl-2-propane sulfonique obtenus à l'issue de cette étape de lavage sont avantageusement isolés de la solution de lavage, par exemple, par une seconde étape de séparation liquide/solide optionnelle illustrée sur la figure 1.

Les cristaux obtenus après cette seconde étape de séparation solide/liquide peuvent être utilisés tels quels ou bien séchés.

Cependant, préférentiellement, les cristaux d'acide acrylamido-2-méthyl-2-propane sulfonique ne sont pas

séchés après la seconde étape de séparation liquide/solide.

La solution de lavage récupérée après la seconde étape de séparation liquide/solide contient principalement de l'eau et de l'acide acrylamido-2-méthyl-2-propane sulfonique solubilisé, et peut minoritairement contenir au moins un solvant organique.

Selon un mode particulier de l'invention, illustré sur la figure 1, la solution de lavage récupérée après la seconde étape de séparation liquide/solide peut être recyclée partiellement ou totalement dans la pré-étape si une telle pré-étape existe, avec ou sans étape de traitement préalable.

Selon un mode particulier de l'invention, illustré sur la figure 1, la solution de lavage récupérée après la seconde étape de séparation liquide/solide peut être recyclée partiellement ou totalement dans l'étape 1), avec ou sans étape de traitement préalable.

Selon un autre mode particulier de l'invention, illustré sur la figure 1, la solution de lavage récupérée après la seconde étape de séparation liquide/solide peut être recyclée partiellement ou totalement pour préparer un sel d'acide d'acrylamido-2-méthyl-2-propane sulfonique, avec ou sans étape de traitement préalable.

Selon un autre mode particulier de l'invention, illustré sur la figure 1, la solution de lavage récupérée après la seconde étape de séparation liquide/solide peut être recyclée partiellement ou totalement pour laver les cristaux d'acide acrylamido-2-méthyl-2-propane sulfonique obtenus après la distillation, avec ou sans étape de traitement préalable.

L'opération de lavage peut être réalisée de manière discontinue (en lot ou « batch ») ou continue. Préférentiellement elle est réalisée en continu.

L'opération de lavage peut être réalisée plusieurs fois successivement si la pureté des cristaux d'acide acrylamido-2-méthyl-2-propane sulfonique n'est pas suffisante.

***Séchage des cristaux d'acide acrylamido-2-méthyl-2-propane sulfonique (optionnel)***

Lors d'une étape optionnelle, illustrée sur la figure 1, les cristaux d'acide acrylamido-2-méthyl-2-propane sulfonique obtenus après l'étape 2) de séparation solide/liquide ou bien obtenus après l'étape de lavage de cristaux, peuvent être utilisés tels quels ou bien séchés. A titre d'exemple et de manière non limitative, le séchage peut être effectué par toute technologie de séchage que ce soit par convection, par conduction ou par rayonnement (séchage à lit fluidisé, séchage lit traversé, séchage sur bande convoyeuse, séchage micro-onde, séchage par rayonnement haute fréquence, infra rouge, séchage par atomisation...).

L'étape optionnelle de séchage peut être réalisée à pression atmosphérique ou bien sous vide.

L'étape optionnelle de séchage peut être réalisée de manière discontinue (en lot ou « batch ») ou continue. Préférentiellement elle est réalisée en continu.

Après l'étape 2) de séparation liquide/solide ou la seconde étape de séparation liquide/solide, les cristaux d'acide acrylamido-2-méthyl-2-propane sulfonique ne sont cependant préférentiellement pas séchés.

Dans un mode particulier de l'invention, il est possible de réaliser les étapes de lavage et de séchage successivement.

Le procédé selon l'invention permet d'obtenir des cristaux d'acide acrylamido-2-méthyl-2-propane sulfonique d'une très grande pureté. Les cristaux d'acide acrylamido-2-méthyl-2-propane sulfonique ainsi isolés ont généralement un taux de pureté compris entre 99,90 et 99,99%, préférentiellement supérieur à 99,95.

Un autre avantage du procédé selon l'invention est l'obtention de cristaux d'acide acrylamido-2-méthyl-2-propane sulfonique ayant un très faible taux d'impuretés, typiquement de type acide 2-méthyl-2-propenyl-sulfonique (IBSA) et acide 2-méthylidene-1,3- propylenedisulfonique (IBDSA), c'est-à-dire une faible quantité de ces impuretés par rapport à la quantité en poids d'ATBS obtenue, ces impuretés pouvant affecter fortement la polymérisation au-delà d'une certaine concentration. Généralement, les cristaux obtenus selon l'invention ont un taux d'IBSA et d'IBDSA inférieur à 100 ppm en poids, préférentiellement un taux inférieur à 50 ppm, encore plus préférentiellement un taux inférieur à 20 ppm par composé. Les autres impuretés que l'on retrouve communément lors de la synthèse d'ATBS sont le tertiobutylacrylamide, l'acrylamide et l'acrylonitrile.

Le taux de pureté et les quantités d'IBDSA et d'IBSA dans les cristaux d'acide 2-acrylamido-2-méthylpropane sulfonique peuvent être mesurés par HPLC (chromatographie en phase liquide à haute performance) selon les conditions suivantes :

- Colonne ODS-3 (GL Science®) ;
- Phase mobile : eau avec 0,03% acide trifluoroacétique / Acétonitrile (ratio massique 90/10) ;
- Débit phase mobile : 0,8ml/minute ;
- Longueur d'onde de détection : 200nm.

L'acide acrylamido-2-méthyl-2-propane sulfonique obtenu peut être sous forme de poudre fine ou mis en forme de manière contrôlée par un procédé tel que la compaction, ou la granulation, ou l'extrusion.

Les cristaux d'acide acrylamido-2-méthyl-2-propane sulfonique obtenus par le procédé selon l'invention peuvent être redissous et circuler de nouveau dans le procédé selon l'invention pour améliorer leur pureté.

***Préparation d'un sel ou d'une solution de sels d'acide acrylamido-2-méthyl-2-propane sulfonique (optionnelle)***

Un autre aspect de l'invention comprendre l'utilisation des cristaux de l'acide acrylamido-2-méthyl-2-propane sulfonique obtenus selon le procédé de l'invention pour la fabrication d'une solution aqueuse de sels d'acrylamido-2-méthyl-2-propane sulfonate.

Les sels d'acrylamido-2-méthyl-2-propane sulfonate sont obtenus par mise en contact et mélange d'une solution aqueuse d'acide acrylamido-2-méthyl-2-propane sulfonique avec au moins un composé de neutralisation choisi parmi les hydroxydes de métal alcalin ou alcalino-terreux, les oxydes de métal alcalin ou alcalino-terreux, l'ammoniaque, les amines de formule suivante $NR_1R_2R_3$ ou les carbonates de métal alcalin ou alcalino-terreux.

Lorsque le composé est un hydroxyde de métal alcalin ou alcalino-terreux, il peut être choisi parmi l'hydroxyde de sodium, l'hydroxyde de potassium, l'hydroxyde de lithium, l'hydroxyde de magnésium et l'hydroxyde de calcium.

Lorsque le composé est un oxyde de métal alcalino-terreux, il peut être choisi parmi l'oxyde de calcium et l'oxyde de magnésium.

Lorsque le composé est une amine de formule $NR_1R_2R_3$, $R_1$, $R_2$ et $R_3$ sont indépendamment un atome d'hydrogène ou une chaîne carbonée contenant de 1 à 22 carbones, avantageusement une chaîne linéaire, $R_1$, $R_2$ et $R_3$ n'étant pas simultanément un atome d'hydrogène. De manière générale, l'ammoniaque ($NH_3$) est préférée aux amines de formule $NR_1R_2R_3$.

Il est possible d'introduire au moins un inhibiteur de polymérisation pendant le procédé de préparation du sel de l'acide acrylamido-2-méthyl-2-propane sulfonique. Cet inhibiteur peut être choisi de manière non limitative parmi l'hydroquinone, le paraméthoxyphénol, le phénotiazine, le 2,2,6,6-tétraméthyl(pipéridin-1-yl)oxyl, le 4-hydroxy-2,2,6,6-tétraméthylpipéridin-1-oxyl, les dérivés de phénylène diamines, ou leurs mélanges.

Préférentiellement, l'inhibiteur est le paraméthoxyphénol.

***Polymères d'acide acrylamido-2-méthyl-2-propane sulfonique***

La demande divulgue également un polymère obtenu à partir de cristaux d'acide acrylamido-2-méthyl-2-propane sulfonique ou de ses sels, obtenus selon le procédé de l'invention.

Le polymère peut être un copolymère comprenant l'acide acrylamido-2-méthyl-2-propane sulfonique obtenu selon le procédé de l'invention, et au moins un monomère hydrosoluble.

Un (co)polymère désigne un homopolymère d'ATBS (forme acide et/ou salifiée) ou un copolymère d'ATBS (forme acide et/ou salifiée) et d'au moins un autre type de monomère.

Le monomère hydrosoluble peut être un monomère non-ionique pouvant notamment être choisi dans le groupe comprenant les monomères vinyliques solubles dans l'eau, et particulièrement l'acrylamide ; le N-isopropylacrylamide ; le N,N-diméthylacrylamide ; la N-vinylformamide ; l'acryloyl morpholine ; le N,N-diéthyle acrylamide ; le N-tert-butyl acrylamide ; le N-tert-octylacrylamide ; la N-vinylpyrrolidone ; la N-vinyl caprolactame ; la N-vinyl-imidazole, l'hydroxyéthyl méthacrylamide, l'hydroxypropylacrylate, l'isoprenol et la diacétone acrylamide. De manière avantageuse, le monomère non-ionique est l'acrylamide.

Le monomère hydrosoluble peut également être choisi dans le groupe des monomères anioniques. Le ou les monomères anioniques pouvant être utilisés dans le cadre de l'invention peuvent être choisis dans un large groupe. Ces monomères peuvent présenter des fonctions vinyliques notamment acryliques, maléiques, fumariques, maloniques, itaconiques, allyliques. Ils peuvent également contenir un groupe carboxylate, phosphonate, phosphate, sulfate, sulfonate, ou un autre groupe à charge anionique. Le monomère anionique peut être sous forme acide ou bien sous forme de sel de métal alcalino-terreux, de sel de métal alcalin ou de sel d'ammonium. Des exemples de monomères convenables comprennent l'acide acrylique ; l'acide méthacrylique ; l'acide itaconique ; l'acide crotonique ; l'acide maléique ; l'acide fumarique ; les monomères de type acide fort présentant par exemple une fonction de type acide sulfonique ou acide phosphonique, tels que l'acide vinylsulfonique, l'acide vinylphosphonique, l'acide allylsulfonique, l'acide méthallylsulfonique, l'acide 2-méthylidenepropane-1,3-disulfonique, le 2-sulfoéthylméthacrylate, le sulfopropylacrylate l'acide allylphosphonique, l'acide styrène sulfonique, l'acide 3-allyloxy-2-hydroxypropane sulfonique ; et les sels hydrosolubles de ces monomères comme leurs sels de métaux alcalins, de métaux alcalino-terreux, ou d'ammonium.

Le monomère hydrosoluble peut être un monomère cationique de type acrylamide, acrylique, vinylique, allylique ou maléique possédant une fonction amine ou ammonium quaternaire. On peut citer, en particulier et de façon non limitative, l'acrylate de diméthylaminoéthyl (ADAME), et le méthacrylate de diméthylaminoéthyle (MADAME) quaternisés ou salifiés, le chlorure de diméthyldiallylammonium (DADMAC), le chlorure d'acrylamido propyltriméthyl ammonium (APTAC) et le chlorure de méthacrylamido propyltriméthyl ammonium (MAPTAC).

Le monomère hydrosoluble peut être un monomère zwitterionique tel que les dérivés ayant un motif acrylamide,

acrylique, vinylique, allylique ou maléique, et possédant une fonction amine ou ammonium quaternaire et une fonction acide de type carboxylique (ou carboxylate), sulfonique (ou sulfonate) ou phosphorique (ou phosphate). On peut citer, en particulier et de façon non limitative les dérivés de l'acrylate de diméthylaminoéthyl, tel que le 2-((2-(acryloyloxy)éthyl) diméthylammonio) éthane-1-sulfonate, le 3-((2-(acryloyloxy)éthyl) diméthylammonio) propane-1-sulfonate, le 4-((2-(acryloyloxy)éthyl) diméthylammonio) butane-1-sulfonate, le [2-(acryloyloxy)éthyl] (diméthylammonio) acétate, les dérivés du méthacrylate de diméthylaminoéthyle tel que le 2-((2-(méthacryloyloxy) éthyl) diméthylammonio) éthane-1-sulfonate, le 3-((2-(méthacryloyloxy) éthyl) diméthylammonio) propane-1-sulfonate, le 4-((2-(méthacryloyloxy) éthyl) diméthylammonio) butane-1-sulfonate, le [2-(méthacryloyloxy)éthyl] (diméthylammonio) acétate, les dérivés du diméthylamino propylacrylamide tel que le 2-((3-acrylamidopropyl) diméthylammonio) éthane-1-sulfonate, le 3-((3-acrylamidopropyl) diméthylammonio) propane-1-sulfonate, le 4-((3-acrylamidopropyl) diméthylammonio) butane-1-sulfonate, le [3-(acryloyloxy) propyl] (diméthylammonio) acétate, les dérivés du diméthylamino propyl méthylacrylamide tel que le 2-((3-méthacrylamidopropyl) diméthylammonio) éthane-1-sulfonate, le 3-((3-méthacrylamidopropyl) diméthylammonio) propane-1-sulfonate, le 4-((3-méthacrylamidopropyl) diméthylammonio) butane-1-sulfonate et le [3-(méthacryloyloxy)propyl] (diméthylammonio) acétate.

Le (co)polymère peut avoir une structure linéaire, branchée, réticulée, en forme d'étoile (ou « star ») ou en forme de peigne (ou « comb »). Ces structures peuvent être obtenues par sélection de l'amorceur, de l'agent de transfert, de la technique de polymérisation telle que la polymérisation radicalaire contrôlée dite RAFT (transfert de chaîne réversible par addition-fragmentation, de l'anglais « reversible-addition fragmentation chain transfer »), NMP (polymérisation en présence de nitroxydes, de l'anglais « Nitroxide Mediated Polymerization ») ou ATRP (polymérisation radicalaire par transfert d'atomes, de l'anglais « Atom Transfert Radical Polymerization »), de l'incorporation de monomères structuraux, de la concentration.

De manière générale, l'obtention du (co)polymère ne nécessite pas de procédé de polymérisation particulier. En effet, il peut être obtenu selon toutes les techniques de polymérisation bien connues par la personne de métier. Il peut notamment s'agir de polymérisation en solution ; polymérisation en gel ; polymérisation par précipitation ; polymérisation en émulsion (aqueuse ou inverse) ; polymérisation en suspension ; polymérisation par extrusion réactive ; ou de polymérisation micellaire.

Le (co)polymère peut être post hydrolysé. La post hydrolyse est la réaction d'hydrolyse du (co)polymère après polymérisation. Cette étape consiste en la réaction des groupes fonctionnels hydrolysables des monomères non ioniques, tel que les fonctions amide ou ester, avec une base. Durant cette étape de post-hydrolyse du copolymère, le nombre de fonction acide carboxylique augmente. En effet, la réaction entre la base et les fonctions amides ou esters présentes dans le copolymère produit des groupes carboxylates.

Le (co)polymère peut se présenter sous forme liquide, gel ou solide lorsque sa préparation inclut une étape de séchage tel que le séchage par pulvérisation (ou « spray drying »), le séchage et granulation par pulvérisation (ou « spray granulation »), le séchage sur tambour, le séchage par rayonnement électromagnétique (micro-ondes, haute fréquence) ou encore le séchage en lit fluidisé.

Le (co)polymère peut avoir un poids moléculaire compris entre 10.000 et 30 millions de daltons. Il peut être un dispersant, un floculant ou un superabsorbant.

Le (co)polymère contient préférentiellement au moins 1 mol% d'acide acrylamido-2-méthyl-2-propane sulfonique obtenu selon le procédé de l'invention, plus préférentiellement au moins 5 mol%, encore plus préférentiellement au moins 10 mol%, encore plus préférentiellement au moins 30 mol%, et encore plus préférentiellement au moins 50 mol%.

Le polymère obtenu à partir de cristaux d'acide acrylamido-2-méthyl-2-propane sulfonique et/ou de ses sels, obtenus selon le procédé de l'invention, peut être utilisé dans la récupération du pétrole et du gaz, dans le traitement de l'eau, dans le traitement des boues, dans la fabrication du papier, dans la construction, dans l'industrie minière, dans la formulation de produits cosmétiques, dans la formulation de détergents, dans la fabrication du textile, ou dans l'agriculture.

Les procédés de récupération de pétrole et de gaz sont généralement des traitements de formations souterraines dans lesquels un polymère est utilisé pour augmenter la viscosité du fluide d'injection aqueux et/ou pour réduire le niveau de résistance au frottement qui se produit lors de l'injection dudit fluide dans une formation souterraine, ou encore pour boucher ponctuellement ou définitivement une partie de la formation souterraine.

Ces traitements souterrains comprennent, sans toutefois s'y limiter, les opérations de forage, les traitements de stimulation tels que les opérations de fracturation, les opérations de complétion et le procédé amélioré de récupération du pétrole par balayage avec une solution de polymères.

Le polymère obtenu à partir de cristaux d'acide acrylamido-2-méthyl-2-propane sulfonique obtenus selon le procédé de l'invention peut être utilisé notamment comme floculant, dispersant, agent épaississant, agent absorbant ou agent réducteur de friction.

L'invention et les avantages qui en découlent ressortiront mieux des exemples suivants donnés afin d'illustrer l'invention, et non de manière limitative.

**Exemples**

L'ensemble des exemples a été réalisé à partir de la même source d'acide acrylamido-2-méthyl-2-propane sulfonique. 700 Kg de granulés d'acide acrylamido-2-méthyl-2-propane sulfonique sont dissous dans 500 Kg d'eau de manière continue dans un réacteur agité. Cet acide acrylamido-2-méthyl-2-propane sulfonique présente une pureté de 99,8 % et comporte 300 ppm d'IBDSA et 500 ppm d'IBSA.

**Exemple 1** - **Comparaison continu par rapport à un lot (« Batch »)**

**1.1** - Dans le cadre de l'invention, la solution d'acide acrylamido-2-méthyl-2-propane sulfonique alimente en continu un évaporateur vertical à couche mince, avec une surface de contact d'un mètre carré, à un débit de 600 $Kg.h^{-1}$. Le temps de résidence est de 34 secondes. L'évaporateur vertical à couche mince est chauffé par une eau à 80°C et la pression de distillation est de 30 mbar. Le débit évaporatoire mesuré est de 72 $Kg/h/m^2$. La suspension d'acide acrylamido-2-méthyl-2-propane sulfonique obtenue après distillation, passe ensuite en continu dans une essoreuse horizontale à panier perforé, ayant une vitesse de rotation de 800 tours par minute, avant d'être analysée. La pureté des cristaux d'acide acrylamido-2-méthyl-2-propane sulfonique obtenue est de 99,9667 % et comporte 44 ppm d'IBDSA et 88 ppm d'IBSA. La productivité en cristaux d'acide acrylamido-2-méthyl-2-propane sulfonique est mesurée à 100 $Kg.h^{-1}$.

Une étape de lavage avec une solution d'eau est réalisée sur le gâteau d'acide acrylamido-2-méthyl-2-propane sulfonique obtenu après passage dans l'essoreuse horizontale à panier perforé. La pureté des cristaux d'acide acrylamido-2-méthyl-2-propane sulfonique obtenue est de 99,9692 % et ces cristaux comprennent 36 ppm d'IBDSA et 72ppm d'IBSA. La productivité en cristaux d'acide acrylamido-2-méthyl-2-propane sulfonique est mesurée à 80 $Kg.h^{-1}$.

**1.2** - La même solution d'acide acrylamido-2-méthyl-2-propane sulfonique que celle de l'exemple 1.1 est préparée avec ajout d'un inhibiteur de polymérisation (340 g de paraméthoxyphénol). La distillation de ladite solution est réalisée dans un réacteur agité (« batch »). Le réacteur dispose d'une double enveloppe ayant une surface de contact de 4,6 $m^2$. La double enveloppe est parcourue par une eau à 80°C. Le réacteur est mis sous vide avec une pression de 30 mbar. Le débit évaporatoire mesuré est de 15 $Kg/h/m^2$. Après 24h, la suspension d'acide acrylamido-2-méthyl-2-propane sulfonique obtenue passe dans une essoreuse horizontale à panier perforé ayant une vitesse de rotation de 800 tours par minute, avant d'être analysée. La pureté des cristaux d'acide acrylamido-2-méthyl-2-propane sulfonique obtenue est de 99,9 % et ces cristaux comprennent 233 ppm d'IBDSA et 466 ppm d'IBSA. La productivité en cristaux d'acide acrylamido-2-méthyl-2-propane sulfonique est mesurée à 20 $Kg.h^{-1}$.

**1.3** - La même procédure que l'exemple 1.2 est suivi à l'exception de l'ajout d'inhibiteur de polymérisation. Après 24h de distillation dans les conditions décrites ci-dessus, la solution d'acide acrylamido-2-méthyl-2-propane sulfonique est partiellement gélifiée. Aucun cristal d'acide acrylamido-2-méthyl-2-propane sulfonique ne peut être filtré.

**1.4** - Un procédé de purification classique avec dissolution dans de l'acide acétique et recristallisation lente a été réalisé. 150 g d'acide acrylamido-2-méthyl-2-propane sulfonique utilisé dans les exemples précédents sont dissous sous agitation dans 500 g d'acide acétique à une température de 90°C. Le mélange est laissé sous agitation durant 1h après la fin de la dissolution des cristaux. La solution est laissée à refroidir pendant 2h à 5°C en dessous de la température de cristallisation de l'acide acrylamido-2-méthyl-2-propane sulfonique. Après une séparation liquide/solide, les cristaux sont mis à sécher dans un four pendant 6h.

Les résultats des différents exemples 1.1-1.4 sont regroupés dans le Tableau 1.

[Tableau 1]

| Exemples | Pression (mbar) | Pureté (%) | IBDSA (ppm) | IBSA (ppm) | Productivité ($Kg.h^{-1}$) |
|---|---|---|---|---|---|
| Solution ATBS de référence | | 99,8 | 300 | 500 | - |
| 1.1 (invention) | 30 | 99,9667 | 44 | 88 | 100 |
| 1.1 avec lavage (invention) | 30 | 99,9692 | 36 | 72 | 80 |
| 1.2 (comparatif, « batch ») | 30 | 99,90 | 233 | 466 | 20 |
| 1.3 (comparatif, « batch ») | - | - | - | - | - |
| 1.4 (comparatif) | - | 99,89 | 159 | 298 | 33 |

**Tableau 1 : Résultats relatifs aux exemples 1.1 à 1.4.**

Nous pouvons observer que le procédé de l'invention permet d'avoir une meilleure productivité que celle d'un

procédé « batch » classique : la productivité est multipliée par cinq. Cela permet d'améliorer la pureté des cristaux d'acide acrylamido-2-méthyl-2-propane sulfonique ainsi que de réduire la proportion d'IBDSA et d'IBSA dans ces cristaux.

Avec une étape de lavage supplémentaire, la quantité d'IBDSA et d'IBSA peut encore être réduite, tout en maintenant une productivité multipliée par quatre par rapport celle d'un procédé « batch ».

Le procédé de l'invention présente donc de meilleures performances que celles d'un procédé « batch », même en l'absence d'inhibiteur de polymérisation. Comme nous pouvons le voir à l'exemple 1.3, sans inhibiteur de polymérisation, l'acide acrylamido-2-méthyl-2-propane auto-polymérise dans le procédé « batch » alors que cela ne se produit pas dans le cas du procédé selon l'invention.

Le procédé de l'invention permet l'obtention de cristaux avec des taux d'IBDSA et IBSA plus faibles, une pureté supérieure et une productivité plus importante par rapport à un procédé de purification classiquement utilisé pour purifier l'acide acrylamido-2-méthyl-2-propane avec une cristallisation lente dans un solvant (exemple 1.4).

**Exemple 2 - Influence de la pression**

L'exemple 1.1 a été reproduit sans l'étape de lavage, à différentes pressions (exemples 2.1 à 2.3). Les résultats sont résumés dans le tableau 2.

[Tableau 2]

| Exemples | Pression (mbar) | Pureté (%) | IBDSA (ppm) | IBSA (ppm) | Productivité ($kg.h^{-1}$) |
|---|---|---|---|---|---|
| Solution ATBS de référence | | 99,8 | 300 | 500 | - |
| 2.1 (invention) | 30 | 99,9667 | 44 | 88 | 100 |
| 2.2 (invention) | 60 | 99,9671 | 10 | 20 | 50 |
| 2.3 (invention) | 300 | 99,963 | 22 | 44 | 10 |

**Tableau 2 : Résultats relatifs aux exemples 2.1 à 2.3.**

Nous pouvons observer que la pression joue un rôle important dans le procédé selon l'invention. En augmentant la pression de distillation, la productivité diminue, alors qu'il est possible de réduire la quantité d'IBDSA et d'IBSA résiduelle. Au-dessus de 300 mbar, la productivité n'est généralement plus intéressante industriellement.

La personne du métier saura ajuster la pression de distillation suivant qu'elle sera intéressée par une plus grande pureté de cristaux d'acide acrylamido-2-méthyl-2-propane ou une plus grande productivité.

**Exemple 3 - Comparaison de solvants**

L'exemple 1.1 a été reproduit sans l'étape de lavage, avec différents solvants (exemples 3.1 à 3.4). Les résultats sont résumés dans le tableau 3.

[Tableau 3]

| Exemples | Solvant | Pureté (%) | IBDSA (ppm) | IBSA (ppm) | Productivité (kg.h-1) |
|---|---|---|---|---|---|
| Solution d'ATBS de référence | | 99,8 | 300 | 500 | - |
| 3.1 (invention) | Eau | 99,9667 | 44 | 88 | 100 |
| 3.2 (invention) | Acétique acide (25) / eau (75) | 99,91 | 154 | 92 | 46 |
| 3.3 (invention) | Acétique acide (8) / eau (92) | 99,9658 | 47 | 94 | 76 |
| 3.4 (invention) | Acrylonitrile (10) / eau (90) | 99,9572 | 43 | 86 | 82 |

**Tableau 3 : résultats relatifs aux exemples 3.1 à 3.4.**

L'utilisation de l'eau comme solvant permet l'obtention de cristaux de meilleure pureté mais également des cristaux contenant moins d'IBDSA et d'IBSA. La productivité est également plus importante pour le procédé utilisant uniquement de l'eau. L'invention permet donc d'avoir un procédé plus productif tout en réduisant l'empreinte environnementale, le coût et les risques liés à l'utilisation, le stockage et la manipulation de solvants.

Le procédé selon l'invention permet l'obtention de cristaux d'acide acrylamido-2-méthyl-2-propane pouvant servir à la préparation de sels d'acide acrylamido-2-méthyl-2-propane et de polymères offrant de meilleures performances que des polymères provenant de cristaux non purifiés selon l'invention.

## Revendications

1. Procédé de purification de l'acide acrylamido-2-méthyl-2-propane sulfonique comprenant les étapes successives suivantes :

   1) préparation d'une suspension de cristaux d'acide acrylamido-2-méthyl-2-propane sulfonique par distillation d'une solution aqueuse d'acide acrylamido-2-méthyl-2-propane sulfonique,
   2) isolation des cristaux d'acide acrylamido-2-méthyl-2-propane sulfonique par séparation solide/liquide de ladite suspension,

   ***caractérisé* en ce que** la distillation se fait en continu et à une pression inférieure à la pression atmosphérique.

2. Procédé selon la revendication 1, ***caractérisé* en ce que** la solution aqueuse d'acide acrylamido-2-méthyl-2-propane sulfonique, préalablement à la distillation, comprend au moins 80% massique d'eau, par rapport à la masse totale des solvants de la solution.

3. Procédé selon l'une des revendications 1 ou 2, ***caractérisé* en ce que** la solution aqueuse comprend au moins un inhibiteur de polymérisation et la quantité d'inhibiteur de polymérisation, par rapport à la quantité de cristaux d'acide acrylamido-2-méthyl-2-propane sulfonique de ladite solution aqueuse, est inférieure à 1% massique.

4. Procédé selon l'une des revendications précédentes, ***caractérisé* en ce que** le solvant distillé issu de l'étape 2) est recyclé au moins partiellement dans la solution aqueuse.

5. Procédé selon l'une des revendications précédentes, ***caractérisé* en ce que** la distillation est effectué dans un dispositif de distillation qui est un évaporateur, de préférence un évaporateur à film mince raclé, ou un évaporateur court trajet, ou un évaporateur à circulation forcée.

6. Procédé selon la revendication 5, ***caractérisé* en ce que** la solution aqueuse d'acide acrylamido-2-méthyl-2-propane sulfonique passe à contre-courant dans le dispositif de distillation.

7. Procédé selon l'une des revendications précédentes, ***caractérisé* en ce que** la durée de distillation est comprise entre 1 et 600 secondes.

8. Procédé selon l'une des revendications précédentes, ***caractérisé* en ce que** la température de la solution d'acide acrylamido-2-méthyl-2-propane sulfonique distillée est comprise entre 5 et 90°C.

9. Procédé selon l'une des revendications précédentes, ***caractérisé* en ce que** la pression lors de la distillation est comprise entre 1 et moins de 1000 mbar absolu.

10. Procédé selon l'une des revendications précédentes, ***caractérisé* en ce que** l'étape d'isolation 2) est réalisée de manière continue.

11. Procédé selon l'une des revendications précédentes, ***caractérisé* en ce que** les cristaux d'acide acrylamido-2-méthyl-2-propane sulfonique obtenus après l'étape 2) sont lavés à l'aide d'au moins une solution de lavage.

12. Procédé selon l'une des revendications précédentes, ***caractérisé* en ce qu'**il comprend une opération de séchage des cristaux d'acide acrylamido-2-méthyl-2-propane sulfonique obtenus après l'étape 2) de séparation solide/liquide

ou bien obtenus après une étape de lavage de cristaux suivie préférentiellement d'une seconde étape de séparation liquide/solide réalisée de manière continue.

**13.** Procédé selon l'une des revendications précédentes, ***caractérisé* en ce que** la concentration en acide acrylamido-2-méthyl-2-propane sulfonique de la solution aqueuse d'acide acrylamido-2-méthyl-2-propane sulfonique distillée à l'étape 1) est comprise entre 10% et la saturation de la solution.

**Patentansprüche**

**1.** Ein Verfahren zur Reinigung von Acrylamido-2-Methyl-2-Propansulfonsäure, das die nachstehenden, aufeinander folgenden Schritte umfasst:

1) Vorbereitung einer Suspension aus Acrylamido-2-Methyl-2-Propansulfonsäure - Kristallen durch Destillation einer wässrigen Lösung aus Acrylamido-2-Methyl-2-Propansulfonsäure,
2) Isolierung der Acrylamido-2-Methyl-2-Propansulfonsäure - Kristalle durch Fest-/ Flüssigtrennung dieser Suspension,

***dadurch gekennzeichnet, dass*** die Destillation kontinuierlich erfolgt und mit einem niedrigeren Druck als der Atmosphärendruck.

**2.** Verfahren nach Anspruch 1, ***dadurch gekennzeichnet, dass*** die wässrige Acrylamido-2-Methyl-2-Propansulfonsäure - Lösung, vor der Destillation, mindestens 80% Masseanteil Wasser bezogen auf die Gesamtmasse der Lösungsmittel der Lösung enthält.

**3.** Verfahren nach einem der Ansprüche 1 oder 2, ***dadurch gekennzeichnet, dass*** die wässrige Lösung mindestens einen Polymerisationsverzögerer enthält und dass die Menge an Polymerisationsverzögerer, bezogen auf die Menge der Acrylamido-2-Methyl-2-Propansulfonsäure - Kristalle dieser wässrigen Lösung unter 1% Masseanteil liegt.

**4.** Verfahren nach einem der vorangehenden Ansprüche, ***dadurch gekennzeichnet, dass das*** destillierte Lösungsmittel aus Schritt 2) zumindest teilweise in der wässrigen Lösung recycelt wird.

**5.** Verfahren nach einem der vorangehenden Ansprüche, ***dadurch gekennzeichnet, dass*** die Destillation in einem Destillierapparat durchgeführt wird, bei dem es sich um einen Verdampfer handelt, vorzugsweise einen Dünnschichtverdampfer oder einen Kurzwegverdampfer oder einen Zwangsumlaufverdampfer.

**6.** Verfahren nach Anspruch 5, ***dadurch gekennzeichnet, dass*** die wässrige Acrylamido-2-Methyl-2-Propansulfonsäure - Lösung gegen die Strömung durch den Destillierapparat fließt.

**7.** Verfahren nach einem der vorangehenden Ansprüche, ***dadurch gekennzeichnet, dass*** die Destillierdauer zwischen 1 und 600 Sekunden beträgt.

**8.** Verfahren nach einem der vorangehenden Ansprüche, ***dadurch gekennzeichnet, dass*** die Temperatur der destillierten Acrylamido-2-Methyl-2-Propansulfonsäure- Lösung zwischen 5 und 90°C liegt.

**9.** Verfahren nach einem der vorangehenden Ansprüche, ***dadurch gekennzeichnet, dass*** der Druck bei der Destillation zwischen 1 und mindestens 1000 mbar absolut beträgt.

**10.** Verfahren nach einem der vorangehenden Ansprüche, ***dadurch gekennzeichnet, dass*** der Schritt der Isolierung 2) kontinuierlich durchgeführt wird.

**11.** Verfahren nach einem der vorangehenden Ansprüche, ***dadurch gekennzeichnet, dass*** die nach Schritt 2) erhaltenen Acrylamido-2-Methyl-2-Propansulfonsäure - Kristalle mit mindestens einer Waschlösung gewaschen werden.

**12.** Verfahren nach einem der vorangehenden Ansprüche, ***dadurch gekennzeichnet, dass*** es einen Vorgang der Trocknung der nach dem Schritt 2) der Fest-/Flüssigtrennung erhaltenen oder aber der nach einem Schritt des Waschens der Kristalle, vorzugsweise gefolgt von einem zweiten Schritt der Fest-/Flüssigtrennung, die kontinuierlich durchgeführt wird, erhaltenen Acrylamido-2-Methyl-2-Propansulfonsäure - Kristalle enthält.

**13.** Verfahren nach einem der vorangehenden Ansprüche, ***dadurch gekennzeichnet, dass*** die Konzentration an der in Schritt 1) destillierten Acrylamido-2-Methyl-2-Propansulfonsäure der wässrigen Lösung zwischen 10% und der Sättigung der Lösung liegt.

## Claims

**1.** Method for the purification of acrylamido-2-methyl-2-propanesulphonic acid, comprising the following successive steps:

1) preparation of a suspension of acrylamido-2-methyl-2-propanesulphonic acid crystals by distillation of an aqueous solution of acrylamido-2-methyl-2-propanesulphonic acid,
2) isolation of the acrylamido-2-methyl-2-propanesulphonic acid crystals by solid/liquid separation of said suspension,

***characterised*** **in that** the distillation is carried out continuously and at a pressure below atmospheric pressure.

**2.** Method according to claim 1, ***characterised*** **in that** the aqueous solution of acrylamido-2-methyl-2-propanesulphonic acid, prior to the distillation, comprises at least 80% by mass of water, based on the total mass of the solvents of the solution.

**3.** Method according to one of claims 1 or 2, ***characterised*** **in that** the aqueous solution comprises at least one polymerisation inhibitor and the amount of polymerisation inhibitor, relative to the amount of acrylamido-2-methyl-2-propanesulphonic acid crystals of said aqueous solution, is less than 1% by mass.

**4.** Method according to one of the preceding claims, ***characterised*** **in that** the distilled solvent resulting from step 2) is recycled at least partially into the aqueous solution.

**5.** Method according to one of the preceding claims, ***characterised*** **in that** the distillation is carried out in a distillation device which is an evaporator, preferably a scraped thin film evaporator, or a short path evaporator, or a forced circulation evaporator.

**6.** Method according to claim 5, ***characterised*** **in that** the aqueous solution of acrylamido-2-methyl-2-propanesulphonic acid is passed in counter-current into the distillation device.

**7.** Method according to one of the preceding claims, ***characterised*** **in that** the distillation time is comprised between 1 and 600 seconds.

**8.** Method according to one of the preceding claims, ***characterised*** **in that** the temperature of the distilled acrylamido-2-methyl-2-propanesulphonic acid solution is between 5 and 90°C.

**9.** Method according to one of the preceding claims, ***characterised*** **in that** the pressure during the distillation is comprised between 1 and less than 1000 mbar absolute.

**10.** Method according to one of the preceding claims, ***characterised*** **in that** the isolating step 2) is carried out continuously.

**11.** Method according to one of the preceding claims, ***characterised*** **in that** the crystals of acrylamido-2-methyl-2-propanesulphonic acid obtained after step 2) are washed with at least one washing solution.

**12.** Method according to one of the preceding claims, ***characterised*** **in that** it comprises a drying operation of the acrylamido-2-methyl-2-propanesulphonic acid crystals obtained after the solid/liquid separation step 2) or else obtained after a crystal washing step followed preferably by a second liquid/solid separation step carried out continuously.

**13.** Method according to one of the preceding claims, ***characterised*** **in that** the concentration of acrylamido-2-methyl-2-propanesulphonic acid in the aqueous solution of acrylamido-2-methyl-2-propanesulphonic acid distilled in step 1) is comprised between 10% and the saturation of the solution.

[Fig. 1]

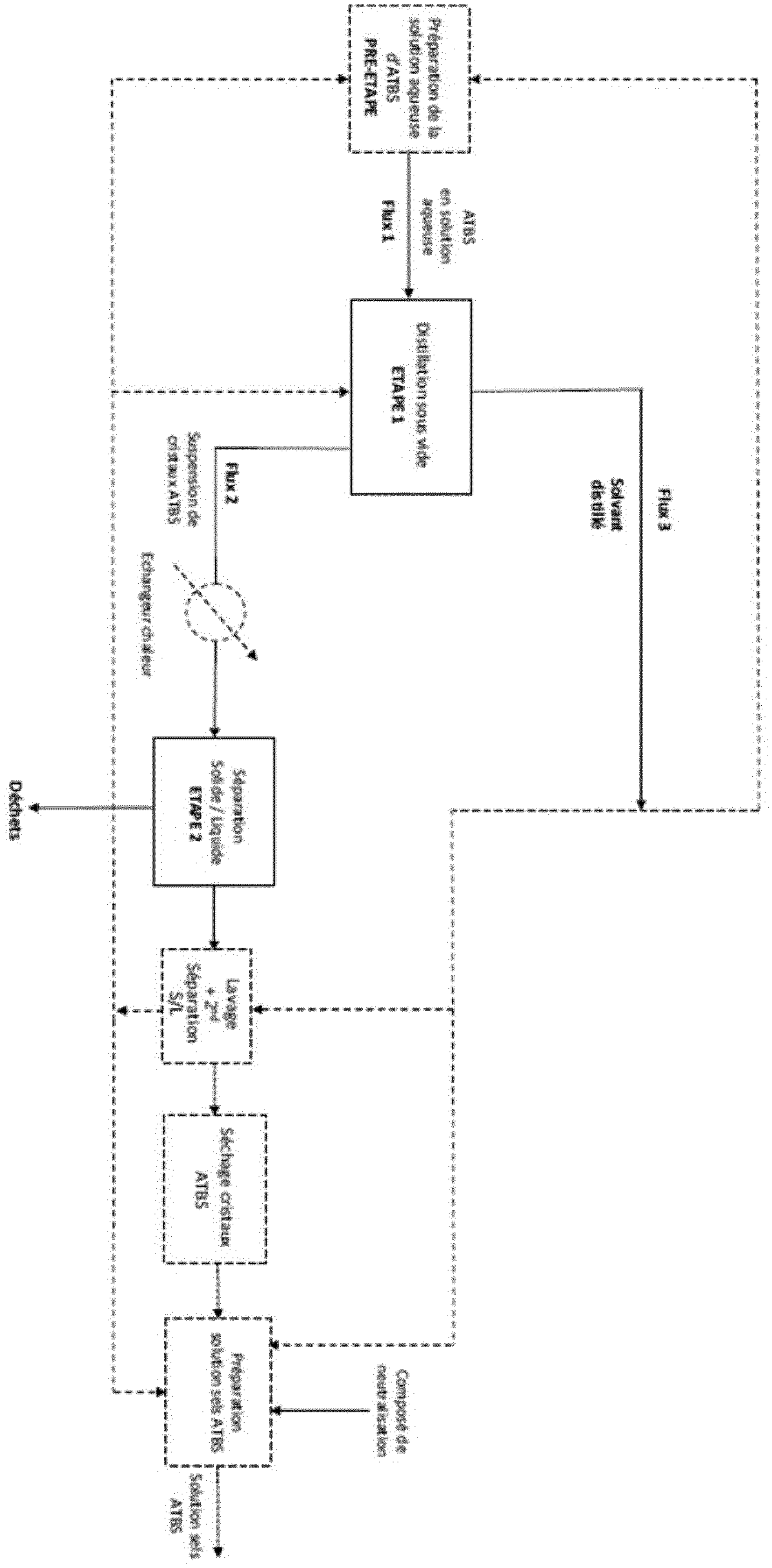
Préparation de la solution aqueuse d'ATBS
PRE-ETAPE
ATBS en solution aqueuse
Flux 1
Distillation sous vide
ETAPE 1
Flux 2
Suspension de cristaux ATBS
Echangeur chaleur
Flux 3
Solvant distillé
Séparation Solide / Liquide
ETAPE 2
Déchets
Lavage + 2nd Séparation S/L
Séchage cristaux ATBS
Préparation solution sels ATBS
Composé de neutralisation
Solution sels ATBS

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6448347 B
- CN 102351744
- WO 2009072480 A
- US 4337215 A
- CN 103664709